# EUROPEAN PATENT APPLICATION

(11) **EP 2 910 183 A1**
(43) Date of publication of application: **26.08.2015**
(21) Application number: 15155493.8
(22) Date of filing: 18.02.2015
(51) Int. Cl.: A61B 5/021, A61B 5/11

(54) **Hemodynamics measurement apparatus and hemodynamics measurement method**

(30) Priority: 25.02.2014 JP 2014034425
(71) Applicant: Nihon Kohden Corporation, Shinjuku-ku Tokyo (JP)
(72) Inventor: Ono, Yoshinobu, Shinjuku-ku, Tokyo (JP); Motogi, Jun, Shinjuku-ku, Tokyo (JP); Nakagiri, Izumi, Shinjuku-ku, Tokyo (JP)
(74) Representative: Grünecker Patent- und Rechtsanwälte PartG mbB

(57) **Abstract**

An exemplary embodiment of a hemodynamics measurement apparatus can include a PWTT measuring unit configured to measure a pulse wave transit time, a body posture measuring unit configured to measure a body posture of a living body, and a data calculation unit configured to calculate data for measuring a hemodynamics value from the measured pulse wave transit time and body posture of the living body. Also, a hemodynamics measurement method can include measuring a pulse wave transit time and a body posture of a living body, and calculating data for measuring a hemodynamics value from the measured pulse wave transit time and body posture of the living body.

## Description

### BACKGROUND

The invention relates to a hemodynamics measurement apparatus and a hemodynamics measurement method for measuring a hemodynamics value for a living body by measuring a PWTT (pulse wave transit time) while changing a position of the body (i.e., changing body posture).

At the outset, blood pressure can be measured so as to evaluate risk of diseases relating to the artery and the heart. In order to increase the reliability of the risk evaluation, it is often helpful to more accurately measure the blood pressure. As disclosed in Japanese Patent No. 3140007 (JP3140007), an apparatus configured to measure both blood pressures of the upper extremity and the lower extremity can be used.

However, according to the apparatus disclosed in JP3140007, cuffs are attached to the upper and lower extremities. For this reason, the blood pressure measurement can be troublesome due to the number and location of cuffs. Also, the apparatus can measure the blood pressure only while the cuffs are attached. Thus, the blood pressure being measured is intermittent and is not continuous.

Therefore, the apparatus disclosed in JP3140007 has at least one drawback in that it intermittently measures accurate blood pressure and is not configured to continuously measure the same. In order to address this drawback, a multifunctional hemadynamometer disclosed in JP-A-8-066377 is configured to measure a PWTT (pulse wave transit time), thereby continuously measuring the blood pressure.

Also, with respect to the technology for attaching the cuff to intermittently measure blood pressure, a head-up tilt test has commonly been used. The head-up tilt test is a test in which changes in blood pressure and pulse are continuously observed at a state of an inclined position so as to see whether the autonomic nerve of a patient is abnormal.

When a posture of the patient is in the state of the inclined position, like the head-up tilt test, it is possible to obtain separate hemodynamics data, which data is typically different from hemodynamics data obtained from the changes in the blood pressure and pulse measured at a sitting state or supine position state.

The inventors thought that when the PWTT is measured at various body postures, a variety of indexes relating to the hemodynamics of the living body, which either could not be obtained or was difficult to obtain in the related art, may be obtained.

According to an aspect of the invention, a hemodynamics measurement apparatus and method can be configured to measure a hemodynamics value for a living body by measuring a PWTT (pulse wave transit time) while changing a body posture.

### SUMMARY

According to a first aspect of the disclosed subject matter, a hemodynamics measurement apparatus can include a PWTT measuring unit, a body posture measuring unit and a data calculation unit.

The PWTT measuring unit can be configured to measure a PWTT (pulse wave transit time). The body posture measuring unit can be configured to measure a body posture of a living body. The data calculation unit can be configured to calculate data for measuring a hemodynamics value from the measured PWTT and body posture.

An example of a hemodynamics measurement method can include measuring a pulse wave transit time and a body posture of a living body, and calculating data for measuring a hemodynamics from the measured pulse wave transit time and body posture of the living body.

According to the exemplary hemodynamics measurement apparatus and hemodynamics measurement method , since the PWTT of the living body is measured while changing the body posture of the living body, it is possible to obtain a variety of indexes relating to the hemodynamics of the living body.

### BRIEF DESCRIPTION OF THE DRAWINGS

FIG. 1 is a block diagram of a hemodynamics measurement apparatus according to an exemplary embodiment.
FIG. 2 is a flowchart illustrating operations of the hemodynamics measurement apparatus of FIG. 1.
FIG. 3 is a view illustrating a PWTT measuring sequence.
FIG. 4 illustrates a specific example of data relating to hemodynamics.

### DETAILED DESCRIPTION OF EMBODIMENTS

Hereinafter, an exemplary embodiment of a hemodynamics measurement apparatus and a hemodynamics measurement method of the invention will be described in detail with reference to the drawings.

### [Configuration of Hemodynamics Measurement Apparatus]

First, a configuration of a hemodynamics measurement apparatus according to an exemplary embodiment of the invention is described.

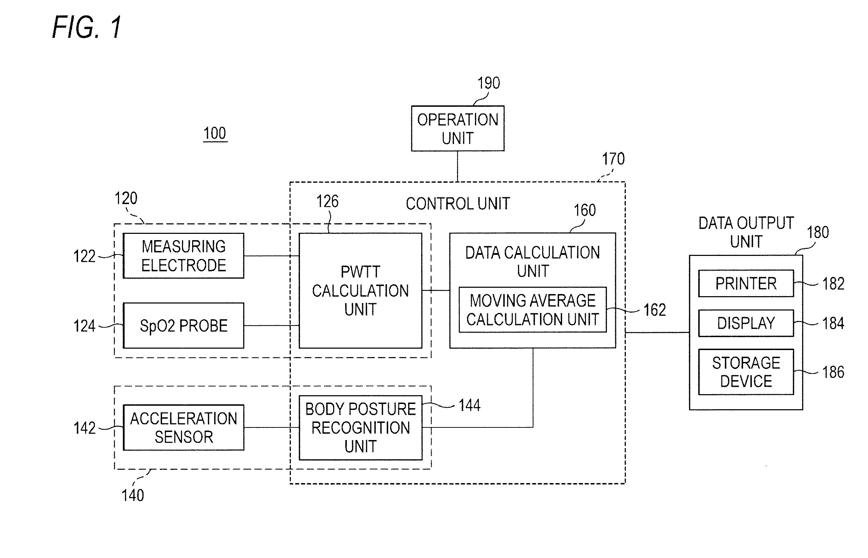

FIG. 1 is a block diagram of a hemodynamics measurement apparatus according to an exemplary embodiment. As shown, a hemodynamics measurement apparatus 100 can include a PWTT measuring unit 120, a body posture measuring unit 140, a data calculation unit 160, a data output unit 180 and an operation unit 190.

The PWTT measuring unit 120 is a unit configured to measure a PWTT (pulse wave transit time). The PWTT measuring unit 120 can include a measuring electrode 122, an SpO2 probe 124 and a PWTT calculation unit 126.

The measuring electrode 122 can be configured to be attached to a body surface of a living body and to acquire an electrocardiographic signal of the living body. The electrocardiographic signal is used for an electrocardiogram drawn by a measurement method such as a Frank lead vector electrocardiogram, a general scalar electrocardiogram, i.e., a standard 12-lead electrocardiogram, a derived lead electrocardiogram, a Holter electrocardiogram, an event electrocardiogram, an exercise electrocardiogram, a monitor electrocardiogram and the like.

The SpO2 probe 124 can be configured to be attached to a distal portion (for example, a finger) of the living body. The SpO2 probe 124 can also be configured to acquire a peripheral pulse wave from a pulsation component relating to the light absorption of the blood.

The PWTT calculation unit 126 can be configured to calculate a PWTT by using the electrocardiographic signal acquired by the measuring electrode 122 and the peripheral pulse wave acquired by the SpO2 probe 124. An exemplary method of calculating the PWTT will be described later.

The body posture measuring unit 140 can be a unit configured to measure a body posture of the living body. The body posture measuring unit 140 can include an acceleration sensor 142 and a body posture recognition unit 144. The acceleration sensor 142 can be configured to be attached to the living body. The acceleration sensor 142 can also be configured to output signals relating to a body posture of the living body such as a dorsal position, a right lateral decubitus position, a left lateral decubitus position and the like and a change of the body posture. The acceleration sensor 142 can, for example, be a three-dimensional acceleration sensor, as shown in the illustrated embodiment. The body posture recognition unit 144 can be configured to recognize the body posture of the living body by using the signals output from the acceleration sensor 142.

The data calculation unit 160 can be configured to calculate data for measuring at least one hemodynamics value associated with the living body from the PWTT measured by the PWTT measuring unit 120 and the body posture of the living body measured by the body posture measuring unit 140. Specifically, the data calculation unit 160 can be configured to input the PWTT calculated by the PWTT calculation unit 126 and the body posture of the living body recognized by the body posture recognition unit 144 and to calculate data for measuring the at least one hemodynamics value associated with the living body from the PWTT and body posture.

The data calculation unit 160 has a moving average calculation unit 162. The moving average calculation unit 162 can be configured to calculate a moving average of the PWTT calculated by the PWTT calculation unit 126. The moving average can be calculated using a running median method. In the meantime, the PWTT calculation unit 126, the body posture recognition unit 144, the data calculation unit 160 and the moving average calculation unit 162 can be embedded in a control unit 170.

The data output unit 180 is a unit configured to output the data calculated in the data calculation unit 160. The data output unit 180 can include a printer 182, a display 184 and a storage device 186. The printer 182 can be configured to print the data calculated in the data calculation unit 160, as a graph. The display 184 can be configured to display the data calculated in the data calculation unit 160, as a graph. The storage device 186 can be configured to store therein the data calculated in the data calculation unit 160.

Meanwhile, the printer 182, the display 184 and the storage device 186 are shown in FIG. 1, as the data output unit 180. However, the data output unit 180 may include only one of these structures. The printer 182 includes a variety of printers such as an inkjet printer, an electrophotographic printer, a sublimation printer and the like. The display 184 includes a variety of displays such as a liquid crystal display, an organic EL display and the like. The storage device 186 includes a variety of storage devices such as a memory card, a compact flash (registered trademark), a multimedia card, a USB memory, a removable hard disk and the like.

The control unit 170 can be connected with the operation unit 190. The operation unit 190 can be configured to set a sampling rate for acquiring the PWTT with respect to the control unit 170. The sampling rate can be set to 4 msec for acquiring the electrocardiographic signal and to 8 msec for acquiring the peripheral pulse wave. Also, the sampling rate can be set to 1 msec or shorter for acquiring the electrocardiographic signal and to 2 msec for acquiring the peripheral pulse wave.

### [Operations of Hemodynamics Measurement Apparatus]

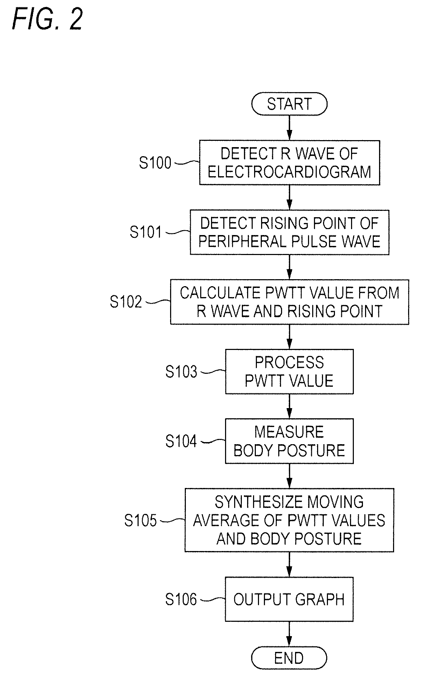

Exemplary operations of the hemodynamics measurement apparatus 100 of the illustrated embodiment will now be described. FIG. 2 is a flowchart illustrating operations of the hemodynamics measurement apparatus of the embodiment shown in FIG. 1. The flowchart of FIG. 2 also illustrates a sequence of a hemodynamics measurement method of the embodiment of FIG. 1.

### <Step S100>

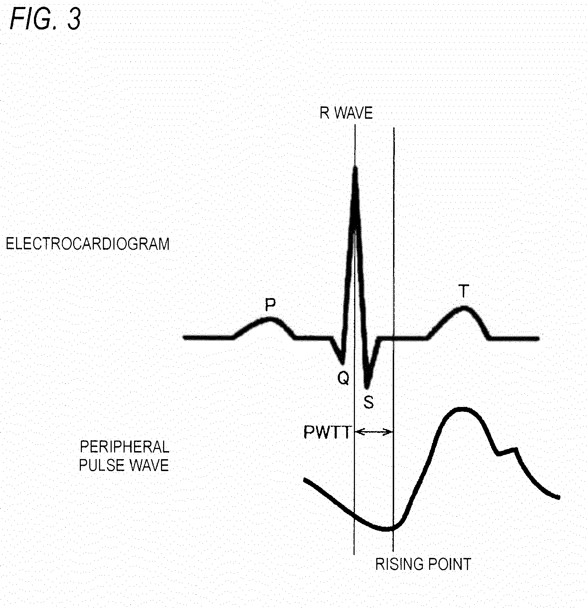

The PWTT calculation unit 126 makes an electrocardiogram as shown in FIG. 3, from the electrocardiographic signal acquired by the measuring electrode 122. The measuring electrode 122 captures a feeble voltage occurring when the heart beats and prepares the electrocardiogram of FIG. 3 by using a variety of measurement methods. As shown, the electrocardiogram has a P wave, a Q wave, an R wave, an S wave, and a T wave. The PWTT calculation unit 126 detects the R wave from the electrocardiogram waveforms.

### <Step S101>

The PWTT calculation unit 126 makes a peripheral pulse wave as shown in FIG. 3, from the pulse wave signal acquired by the SpO2 probe 124. The SpO2 probe 124 captures a change in a light absorption degree (for example, a red light absorption degree) of the blood sent by the beat of the heart, and prepares the peripheral pulse wave of FIG. 3. The PWTT calculation unit 126 detects a rising point of the peripheral pulse wave.

### <Step S102>

The PWTT calculation unit 126 calculates the PWTT value from the detected R wave of the electrocardiogram and the detected rising point of the peripheral pulse wave. The PWTT calculation can be performed as follows. As shown in FIG. 3, the PWTT value indicates time after the R wave of the electrocardiogram is detected until the rising point of the peripheral pulse wave is detected. Therefore, when the time at which a top point of the R wave of the electrocardiogram is detected is denoted with T0 and the time at which the rising point of the peripheral pulse wave is detected is denoted with T1, time of T1-T0 is the PWTT value. In general, when the blood pressure increases, the blood vessel of the living body becomes hard, so that the rising point of the peripheral pulse wave becomes fast. Therefore, the PWTT value becomes a short time. On the other hand, when the blood pressure decreases, the blood vessel of the living body becomes soft, so that the rising point of the peripheral pulse wave becomes late. Therefore, the PWTT value becomes a long time. Hence, it is possible to capture the change in the blood pressure by calculating the PWTT value.

### <Step S103>

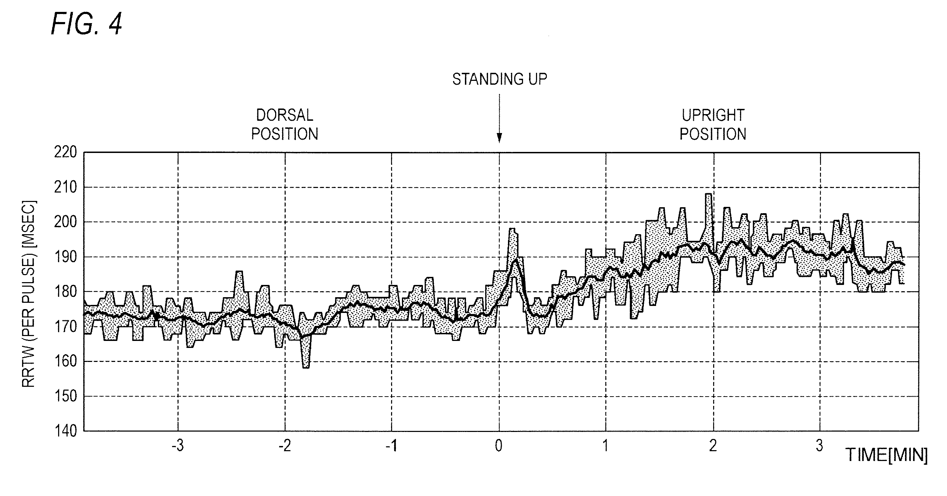

The moving average calculation unit 162 arranges the PWTT values calculated by the PWTT calculation unit 126 for each pulse in chronological order, and calculates an average value of the PWTT values corresponding to 3 pulses before and after the PWTT value of any pulse, respectively, i.e., a total of 7 pulses (the running median method). The moving average calculation unit 162 performs the corresponding processing for all the pulses to calculate a moving average of the PWTT values. A visualized result of the moving average of the PWTT values is data expressing a curve as shown with the solid line in FIG. 4.

### <Step S104>

The body posture recognition unit 144 measures the body posture of the living body, from the signal output by the acceleration sensor 142.

The steps S100 to S04 corresponds to a measuring step of the hemodynamics measurement method of the embodiment of FIG. 1, and can be completed sequentially or in different non-sequential order.

### <Step S105>

The data calculation unit 160 synthesizes both data of the moving average of the PWTT values calculated by the moving average calculation unit 162 and the body posture of the living body measured by the body posture recognition unit 144. A visualized result of the synthesized data is data for measuring the hemodynamics, which expresses a curve as shown in FIG. 4. The data calculation unit 160 outputs the synthesized data to the data output unit 180.

The step S105 corresponds to a calculation step of an exemplary hemodynamics measurement method.

### <Step S106>

The data output unit 180 outputs the data for measuring the hemodynamics, which is obtained by synthesizing the moving average of the PWTT values and the body posture of the living body, as a graph as shown in FIG. 4. When the printer 182 outputs the data, the printer outputs the graph as shown in FIG. 4 on a sheet, and when the display 184 outputs the data, the display outputs the graph as shown in FIG. 4 on a display. When the data output unit outputs the data to the storage device 186, the data output unit stores the original data of the graph shown in FIG. 4 in the storage device 186.

The step S105 corresponds to an output step of an exemplary hemodynamics measurement method.

A vertical axis of FIG. 4 indicates a PWTT value (msec) and a horizontal axis indicates time (min). When the living body stands up, zero (0) is set. -1 is set for one minute before the standing up, -2 is set for two minutes before the standing up, 1 is set for one minute after the standing up and 2 is set for two minutes after the standing up. The curve shown with the solid line indicates the moving average of the PWTT values when the body posture is changed in order of the dorsal position, the standing up and the upright position. It can be seen from FIG. 4 how the moving average of the PWTT values changes depending on the body posture. That is, it is possible to know a correlation between the change in the moving average of the PWTT values and the change in the body posture.

Gray areas drawn around the curve of the moving average of the PWTT values indicate the scattering of the PWTT values (before the calculation of the moving average) calculated by the PWTT calculation unit 126 and are obtained by combining minimum values of the PWTT values with each other and maximum values thereof with each other. It can be seen that the PWTT values are varied within the ranges of the gray areas.

In this illustrated embodiment, as described above, a sampling rate higher than the normally used sampling rate is used so as to acquire the electrocardiographic signal, and the sampling rate higher than the normally used sampling rate is used so as to acquire the peripheral pulse wave.

For this reason, according to this embodiment, it is possible to acquire the PWTT values in a shorter time than in the related art. From the correlation between the PWTT values acquired in a short time and the body posture, it is possible to know many opinions.

For example, it is possible to manage a dose of medication and to change a type of medication by seeing the data for measuring the hemodynamics as shown in FIG. 4, which is output from the printer 182. The reason is that the correlation between the body posture and the PWTT value becomes different depending on a difference of the amount or type of medication.

Also, while examining the apnea syndrome, it is possible to know at which body posture the apnea occurs by seeing the data measuring the hemodynamics as shown in FIG. 4, which is output from the printer 182.

Further, it is also possible to know a progressing degree of the hyperpiesia and a progressing degree of the arteriosclerosis by seeing the data for measuring the hemodynamics as shown in FIG. 4, which is output from the printer 182.

The data for measuring the hemodynamics value(s), which is output by the data calculation unit 160, may be stored in the storage device 186 and may be appropriately analyzed by a dedicated analysis program for each disease to analyze presence of each disease and/or a progressing degree of the disease.

Although the illustrated embodiment of the invention has been described, the illustrated embodiment is just exemplarily provided so as to provide an example of the invention and the scope of the invention is not limited to the illustrated embodiment. The invention can be implemented in a variety of aspects different from the illustrated embodiment without departing from the spirit of the invention.

It will be apparent to those skilled in the art that various modifications and variations can be made in the invention without departing from the spirit or scope of the invention. Thus, it is intended that the invention cover the modifications and variations of the invention provided they come within the scope of the appended claims and their equivalents. All related art references described above are hereby incorporated in their entirety by reference.

## Claims

1. A hemodynamics measurement apparatus comprising:
a PWTT measuring unit configured to measure a pulse wave transit time;
a body posture measuring unit configured to measure a body posture of a living body; and
a data calculation unit configured to receive data from the PWTT measuring unit and body posture measuring unit, and to calculate at least one hemodynamics value from the data representative of the measured pulse wave transit time and body posture of the living body.

2. The hemodynamics measurement apparatus according to claim 1, further comprising:
a data output unit configured to output the at least one hemodynamics value calculated in the data calculation unit.

3. The hemodynamics measurement apparatus according to claim 1 or 2, wherein the PWTT measuring unit includes:
an electrode configured to acquire an electrocardiographic signal;
a probe configured to acquire a pulse wave; and
a PWTT calculation unit configured to calculate the pulse wave transit time from the acquired electrocardiographic signal and pulse wave.

4. The hemodynamics measurement apparatus according to claim 3, wherein a sampling rate for acquiring the electrocardiographic signal is 4 msec or shorter and a sampling rate for acquiring the pulse wave is 8 msec or shorter.

5. The hemodynamics measurement apparatus according to any one of claims 1 to 4, wherein the body posture measuring unit includes:
an acceleration sensor configured to be mounted to the living body and to output a signal relating to the body posture, and
a body posture recognition unit configured to recognize the body posture of the living body by using the signal output from the acceleration sensor.

6. The hemodynamics measurement apparatus according to any one of claims 1 to 5, wherein the data calculation unit includes a moving average calculation unit configured to calculate a moving average of the measured pulse wave transit time, and
wherein the data for measuring the at least one hemodynamics value is calculated from the measured body posture and the calculated moving average of the pulse wave transit time.

7. The hemodynamics measurement apparatus according to claim 1, wherein the data calculation unit is configured to calculate a plurality of different hemodynamics values at an instant in time.

8. A hemodynamics measurement method comprising:
providing a hemodynamics measurement apparatus including a PWTT measuring unit and a body posture measuring unit;
measuring a pulse wave transit time using the PWTT measuring unit;
measuring a body posture of a living body using the body posture measuring unit; and
calculating at least one hemodynamics value from the measured pulse wave transit time and the measured body posture of the living body.

9. The hemodynamics measurement method according to claim 8, wherein measuring the pulse wave transit time includes obtaining an electrocardiographic signal using a sampling rate of 4 msec or shorter for acquiring the electrocardiographic signal, and includes obtaining a pulse wave using a sampling rate of 8 msec or shorter for acquiring the pulse wave.

10. The hemodynamics measurement method according to claim 8 or 9, further comprising providing an output unit and outputting the calculated data to the output unit.

11. The hemodynamics measurement method according to any one of claims 8 to 10, wherein calculating the at least one hemodynamics value further includes calculating a moving average of the measured pulse wave transit time, and
wherein the at least one hemodynamics value is calculated from the measured body posture and the calculated moving average of the pulse wave transit time.

12. The hemodynamics measurement method according to claim 8, wherein calculating at least one hemodynamics value includes calculating a plurality of different hemodynamics values at an instant in time.
